# EUROPÄISCHE PATENTSCHRIFT

(11) **EP 3 308 690 B1**
(45) Veröffentlichungstag und Bekanntmachung des Hinweises auf die Patenterteilung: **28.11.2018**
(21) Anmeldenummer: 17195164.3
(22) Anmeldetag: 06.10.2017
(51) Int. Cl.: A61B 1/00, A61B 1/12, G01M 3/28, A61L 2/24, G01M 3/26

(54) **VERFAHREN ZUM AUFBEREITEN EINES ENDOSKOPS**
METHOD FOR REPROCESSING OF AN ENDOSCOPE
PROCÉDÉ DE RECONDITIONNEMENT D'UN ENDOSCOPE

(30) Priorität: 14.10.2016 DE 102016220137
(43) Veröffentlichungstag der Anmeldung: 18.04.2018
(73) Patentinhaber: OLYMPUS Winter & Ibe GmbH, 22045 Hamburg (DE)
(72) Erfinder: THATE, Henning, 22417 Hamburg (DE)
(74) Vertreter: Seemann & Partner Patentanwälte mbB

(56) Entgegenhaltungen:
- EP-A1- 1 785 150
- EP-A2- 1 779 769
- DE-A1-102012 218 754
- US-A1- 2005 056 081
- US-A1- 2007 238 923

## Beschreibung

Die Erfindung betrifft ein Verfahren zum Aufbereiten eines, insbesondere flexiblen, Endoskops, wobei ein aufzubereitendes Endoskop zur Dichtigkeitsprüfung des Endoskops mit einem Druckluftschlauch druckluftdicht verbunden wird.

Zur Aufbereitung von, insbesondere flexiblen, Endoskopen werden üblicherweise Reinigungs- und Desinfektionsgeräte (RDG-E) eingesetzt, die auf einem chemisch-thermischen Aufbereitungsprozess basieren. Um die Endoskope durch die Reinigungsflüssigkeit nicht zu beschädigen, ist es wichtig, dass die Hülle des Endoskops vollständig intakt ist und somit Reinigungsflüssigkeit nicht in das Innere des Endoskops eindringen kann. Dies wird im RDG-E vor dem ersten Wassereinlauf überprüft, indem das Endoskop über eine entsprechende Schnittstelle auf einen Überdruck aufgepumpt wird. Anschließend wird der Druckabfall überwacht, wobei aus einem Druckabfall auf eine Leckage in dem Endoskop geschlossen werden kann. Bei fehlgeschlagener Prüfung bzw. zu Prozessende, also zum Ende des Reinigungs- und Desinfektionsprozesses, wird das Endoskop wieder teilweise entlüftet. Die Aufbereitung selbst erfolgt bei geringerem Überdruck im Endoskop.

Entsprechende RDG-E-Systeme der OLYMPUS Winter & Ibe GmbH, Hamburg, sind unter der Bezeichnung "ETD" bekannt. Diese Systeme umfassen einen sogenannten "Leak Tester", der den Dichtigkeitstest durchführt. Dazu wird ein Endoskop initial nach Programmstart zur Dichtigkeitsprüfung mit einem Überdruck von beispielsweise 285 mbar gegenüber dem Umgebungsdruck aufgepumpt und der Überdruck nach dem Dichtigkeitstest auf ca. 150 mbar über Umgebungsdruck reduziert. Dieser Druck wird während der Aufbereitung gehalten und kontinuierlich überwacht, auch um das Eindringen von Wasser von außen zu verhindern. Ein entsprechendes Aufbereitungsverfahren mit Dichtigkeitsprüfung ist beispielsweise aus der Druckschrift DE 10 2012 218754 A1 bekannt.

Bei der Verwendung der Endoskope in der Endoskopie treten vereinzelt Schäden auf, bei denen Endoskope von Biopsiekanälen her punktiert werden. Es entsteht eine Mikroperforation, die bei einem durchgeführten Dichtigkeitstest unter Umständen nicht erkannt wird. Bei der nachfolgenden Aufbereitung wird Spül- und Klarwasser mit einem deutlich größeren Druck als 150 mbar durch die zu spülenden Kanäle geleitet. Dadurch kann dann ein Übertrag von Wasser aus der Kanalspülung in den zu schützenden Bereich des Endoskops erfolgen, was zu einer Druckerhöhung im Endoskop über 150 mbar hinaus führt. Der Leak Tester reagiert darauf mit einem Ablassen von Druckluft aus dem Endoskop, um den Überdruck von 150 mbar zu halten. Dadurch kann Wasser über den Weg der Entlüftung in die Verschlauchung eindringen und schlussendlich in den Leak Tester eingetragen werden und diesen schlimmstenfalls zerstören.

Bei der Prüfung der Dichtigkeit von chirurgischen Instrumenten, z.B. Endoskopen, wird das Endoskop mit einer Dichtigkeitstesteinrichtung verbunden, um zu überprüfen, ob die äußere Hülle des Endoskops sowie die Kanäle des Endoskops vollständig intakt sind.

Vor dem ersten Reinigungsvorgang mit einer Flüssigkeit, wie z.B. Wasser, wird hierbei automatisch überprüft, indem die Dichtigkeitstesteinrichtung über einen entsprechenden Anschluss mit dem Endoskop verbunden wird und anschließend auf einen Überdruck aufgepumpt wird. Danach wird der Druckabfall überwacht, um eine Leckage im Endoskop festzustellen.

Sofern in einem undichten Endoskop Wasser vorhanden ist, kann dies dazu führen, dass das eingedrungene Wasser beim Entlüften in das Reinigungs- und Desinfektionsgerät (RDG-E) gelangen kann, wodurch dieses ebenfalls beschädigt wird.

Um die Undichtigkeit zu erkennen, wird vor der Durchführung des Reinigungsvorgangs des Endoskops das Endoskop auf einen Prüfdruck gebracht, typischerweise 275 mbar bis 300 mbar, und dann für eine bestimmte Zeit, z.B. 60 Sekunden, geprüft. Fällt der Druck innerhalb des Endoskops um mehr als einen zuvor festgelegten Betrag, typischerweise zwischen 10 mbar bis 15 mbar, ab, so wird das Endoskop als undicht angesehen, so dass die anschließende Aufbereitung nicht durchgeführt wird.

Darüber hinaus besteht bei den derzeitigen Dichtigkeitsprüfverfahren eine Ungenauigkeit darin, bestimmte Arten von Undichtigkeiten, vor allem bei richtungsabhängigen Undichtigkeiten, diese zuverlässig zu erkennen.

Ausgehend von diesem Stand der Technik besteht die Aufgabe der Erfindung darin, insbesondere bei der Durchführung eines automatischen Dichtigkeitstest in einer Aufbereitungseinrichtung, die Dichtigkeitsprüfung auf einfache Weise zu verbessern.

Gelöst wird diese Aufgabe durch ein Verfahren zum Aufbereiten eines, insbesondere flexiblen, Endoskops, wobei ein aufzubereitendes Endoskop zur Dichtigkeitsprüfung des Endoskops mit einem Druckluftschlauch druckluftdicht verbunden wird, der mit einer Druckluftquelle verbindbar oder verbunden ist, wobei für die Durchführung eines pneumatischen Dichtigkeitstests das Endoskop durch den Druckluftschlauch bis zu einem vorgegebenen oder vorgebbaren Druckniveau mit Druckluft beaufschlagt wird, wobei während der pneumatischen Dichtigkeitsmessung nach Öffnen eines Ablassventils ohne weitere Druckluftzufuhr ein zeitlicher Verlauf des Luftdrucks im Endoskop gemessen wird, wobei die zeitliche Änderung des Luftdrucks am Ablassventil erfasst wird und in Abhängigkeit des zeitlichen Gradienten der zeitlichen Änderung des Luftdrucks die Dichtigkeit des Endoskops oder die Undichtigkeit des Endoskops ermittelt wird.

Die Erfindung beruht auf dem Gedanken, dass bei einem Endoskop vor Durchführung der Aufbereitungsprozesse das Ablassventil des Endoskops eingangsseitig kurz geöffnet wird, um eine Leckage anhand der Geschwindigkeit, mit der der Druck ausgangsseitig (wieder) ansteigt, zu erfassen.

Gemäß dem Stand der Technik wird bei den herkömmlichen Verfahren zur Erkennung einer Leckage z.B. zur Prüfung der Dichtigkeit der Endoskope jeweils der zeitliche Verlauf des Druckausgleichs erfasst. Allerdings können auch bei Endoskopen mit einer hohen Packungsdichte innerhalb des Endoskops über einen längeren Zeitraum von einer Minute und mehr (Druck-) Ausgleichsvorgänge im Inneren der Endoskope stattfinden. Die Zeit für diese Ausgleichsvorgänge entspricht auch etwa der Zeitdauer für die übliche Dichtigkeitsprüfung gemäß dem Stand der Technik. Hierbei wird geprüft, ob der im Anschluss des Endoskops gemessene Druck über den gesamten Zeitraum von beispielsweise einer Minute auch bei intakten Endoskopen mit einer hohen Packungsdichte abfällt, so dass der Vorgang des Druckausgleichs innerhalb der Endoskope hoher Packungsdichte messtechnisch nicht von einer Undichtigkeit zu unterscheiden ist.

Demgegenüber wird gemäß der Erfindung die zeitliche Änderung des Luftdrucks für eine kurze Zeitdauer von weniger als 20 Sekunden ermittelt, so dass in der Abhängigkeit des zeitlichen Gradienten eine einfache und sichere Methode für die Dichtigkeitsprüfung auch von Endoskopen mit einer hohen Packungsdichte bereitgestellt wird. Anhand des zeitlichen Gradienten der zeitlichen Änderung des Luftdrucks wird auch sicher eine Leckage von Endoskopen mit einer hohen Packungsdichte erfasst. Hierbei wird gemäß der Erfindung nach kurzzeitigem Öffnen des Ablassventiles die Geschwindigkeit, mit der der Druck ausgangsseitig aufgrund von internen Druckausgleichsvorgängen im Endoskop und einer dadurch bewirkten Wirkungsumkehr ansteigt, erfasst. Hierbei entspricht die Geschwindigkeit, mit der der Druck ausgangsseitig ansteigt, dem zeitlichen Gradienten der zeitlichen Änderung des Luftdrucks. Aufgrund der hohen Packungsdichte bei Endoskopen erfolgen Druckausgleichsvorgänge, wodurch eine Wirkungsumkehr des Drucks mit einem Anstieg des Drucks beim Ablassen unmittelbar nach Öffnen des Ablassventils erfolgt. Hierbei erfolgt nach dem Druckablassen ein kontinuierlicher Druckanstieg innerhalb des Eigenvolumens des Dichtigkeitsprüfers bis zu einem Maximum.

Erfindungsgemäß ist hierbei bei einem Endoskop mit einer Undichtigkeit die anfängliche Geschwindigkeit, mit der der Druck ausgangsseitig ansteigt, geringer als die Geschwindigkeit und damit der zeitliche Gradient der zeitlichen Änderung des Luftdrucks bei einem dichten Endoskop.

Bei dem erfindungsgemäßen Verfahren wird deshalb ausgehend von einem konstanten Eigenvolumen bzw. Totvolumen der Dichtigkeitsprüfeinrichtung der Druckanstieg nach Öffnen des Ablassventils über einen vorbestimmten, relativ kurzen Zeitraum erfasst.

Dazu ist in einer Weiterbildung des Verfahrens vorgesehen, dass bei Überschreiten eines vorbestimmten Gradientensollwerts für den zeitlichen Gradienten der zeitlichen Änderung des Luftdrucks die Dichtigkeit des Endoskops festgestellt wird. Bei einem intakten, d.h. dichten Endoskop ist der zeitliche Gradient der Änderung des Luftdrucks größer als der anfängliche zeitliche Gradient der Druckänderung bei einem undichten Endoskop.

Hierbei werden vor der Durchführung des Verfahrens für verschiedene Endoskope die jeweiligen spezifischen Gradientensollwerte ermittelt und beispielsweise in einer Datenbank oder einem elektronischen Speicher hinterlegt. Hierbei kann bei der Durchführung der Aufbereitung anhand der hinterlegten spezifischen Gradientensollwerte der jeweilige Gradientensollwert für ein bestimmtes Endoskop oder einen bestimmten Endoskoptyp für die Ermittlung des Dichtigkeitszustands des aufzubereitenden Endoskops verwendet werden.

Darüber hinaus ist in einer vorteilhaften Weiterbildung des Verfahrens vorgesehen, dass bei Unterschreiten eines oder des vorbestimmten Gradientensollwerts für den zeitlichen Gradienten der zeitlichen Änderung des Luftdrucks die Undichtigkeit des Endoskops festgestellt wird.

Außerdem zeichnet sich eine Weiterbildung des Verfahrens dadurch aus, dass vorzugsweise ausschließlich, der zeitliche Anstieg des Drucks nach Öffnen des Ablassventils und/oder dessen zeitliche Änderung erfasst werden.

Insbesondere wird das Ablassventil für eine Zeitdauer von weniger als 20 Sekunden, insbesondere weniger als 10 Sekunden oder 5 Sekunden, geöffnet.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines Reinigungs- und Desinfektionsgeräts;
- Fig. 2: schematisch den Druckverlauf über die Länge des Endoskopschafts bei einem dichten Endoskop und bei einem undichten Endoskop.

In den Zeichnungen sind jeweils gleiche oder gleichartige Elemente und/oder Teile mit denselben Bezugsziffern versehen, so dass von einer erneuten Vorstellung jeweils abgesehen wird.

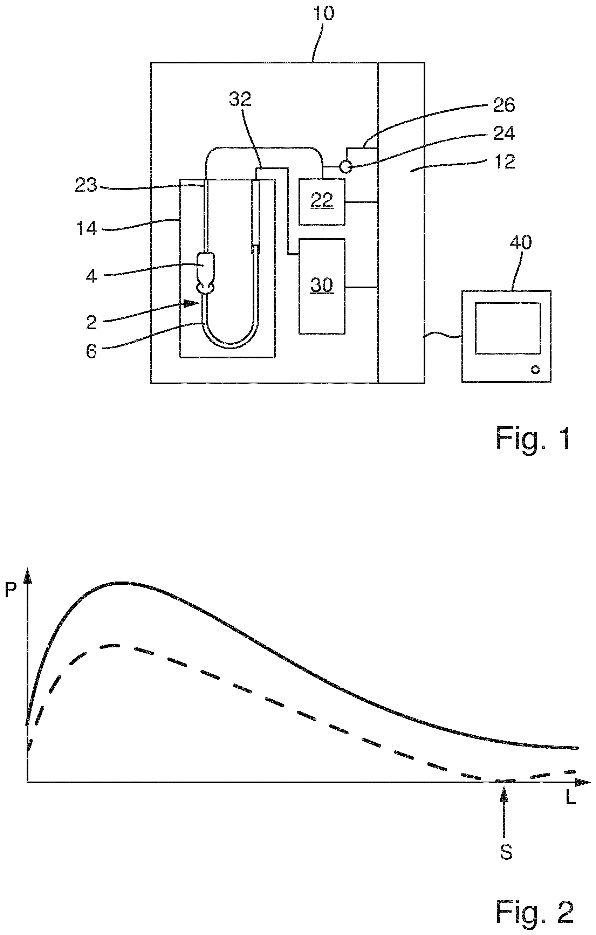

Fig. 1 zeigt eine schematische Darstellung eines Aufbereitungssystems 10, in dessen Reinigungskammer 14 ein flexibles Endoskop 2 angeordnet ist, das bereit ist, gereinigt und desinfiziert zu werden. Das Endoskop 2 umfasst einen Griff 4 sowie einen flexiblen Schaft 6, die beide an Anschlüsse des Aufbereitungssystems 10 angeschlossen sind.

Der Schaft 6 des Endoskops 2 ist über einen Anschluss mit einer Aufbereitungsvorrichtung 30 des Aufbereitungssystems 10 verbunden, während der Handgriff 4 über einen Druckluftschlauch 23 mit einer Druckluftquelle 22, beispielsweise einem Kompressor, mit der Vorrichtung 20 zur Druckluftbeaufschlagung, kurz "Leakage Tester" genannt, verbunden ist. Diese ist, ebenso wie die Aufbereitungsvorrichtung 30, mit einer Steuervorrichtung 12 des Aufbereitungssystems 10 verbunden, die somit auch eine Steuervorrichtung für die Vorrichtung 20 zur Druckluftbeaufschlagung darstellt.

Ausgangs der Druckluftquelle 22 ist ein Luftdrucksensor 24 an den Druckluftschlauch 23 angeschlossen, der den Überdruck der Luft in dem Druckluftschlauch 23 über dem Umgebungsdruck misst und entsprechende Signale über eine Signalleitung 26 ebenfalls an die Steuervorrichtung 12 des Aufbereitungssystems 10 übermittelt. Die Steuervorrichtung 12 ist außerdem mit einer Anzeigevorrichtung 40 verbunden, mittels der die Steuervorrichtung 12 von außen beeinflusst werden kann und umgekehrt Daten aus der Steuervorrichtung 12, beispielsweise Überdruckmessdaten von dem Luftdrucksensor 24 und/oder Prozessdaten, angezeigt werden können.

Die Vorrichtung 20 zur Druckluftbeaufschlagung ist mit einer Druckluftquelle 22 über einen Druckluftschlauch 23 mit einem nur schematisch angedeuteten Endoskop 2 verbunden, wodurch Druckluft in das Endoskop 2 gepumpt wird und danach auch wieder ausgelassen wird.

Mit dem Druckluftschlauch 23 ist ein Luftdrucksensor 24 verbunden, der den Druck der Druckluft im Druckluftschlauch 23 und im Endoskop 2 misst. Der Luftdrucksensor 24 kann alternativ auch am Endoskop 2 direkt messen oder am Ausgang der Druckluftquelle 22.

In Fig. 2 ist schematisch der jeweilige Druckverlauf im Inneren eines dichten Endoskops mit einer durchgezogenen Linie und eines undichten Endoskops mit einer gestrichelten Linie dargestellt, wobei hierbei der schematische Druckverlauf im Inneren der Endoskope direkt nach einer kurzen Öffnung eines Ablassventiles wiedergegeben ist. Erfindungsgemäß wird bei der Prüfung der Dichtigkeit der Endoskope die Geschwindigkeit, mit der der Druck ausgangsseitig nach kurzzeitigem Öffnen des Ablassventiles wieder ansteigt, und damit der zeitliche Gradient der zeitlichen Änderung des Drucks beim Ablassen betrachtet bzw. erfasst, da bei einem dichten Endoskop aufgrund von Druckausgleichsvorgängen der Druck am Ablassventil wieder ansteigt.

Bei dem undichten Endoskop, das eine Leckage aufweist (vgl. gestrichelte Linie), ist die Geschwindigkeit, d.h. der zeitliche Gradient der zeitlichen Änderung des Luftdrucks, geringer, da aufgrund der Leckage und aufgrund der Ausgleichsvorgänge der Druck am Einlassventil bzw. der Druckanstieg geringer ist, da gleichzeitig noch ein Ausgleichsvorgang hin zur an der mit S bezeichneten Leckagestelle stattfindet.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezugszeichenliste

- 2: Endoskop
- 4: Griff
- 6: flexibler Schaft
- 10: Aufbereitungssystem
- 12: Steuereinheit
- 14: Reinigungskammer
- 20: Vorrichtung zur Druckluftbeaufschlagung
- 22: Druckluftquelle
- 23: Druckluftschlauch
- 24: Luftdrucksensor
- 26: Signalleitung
- 30: Aufbereitungsvorrichtung
- 32: Spülschlauch
- 40: Anzeigeeinheit

- S: Leckagestelle

## Patentansprüche

1. Verfahren zum Aufbereiten eines, insbesondere flexiblen, Endoskops (2), wobei ein aufzubereitendes Endoskop (2) zur Dichtigkeitsprüfung des Endoskops (2) mit einem Druckluftschlauch druckluftdicht verbunden wird, der mit einer Druckluftquelle verbindbar oder verbunden ist, wobei für die Durchführung eines pneumatischen Dichtigkeitstests das Endoskop (2) durch den Druckluftschlauch bis zu einem vorgegebenen oder vorgebbaren Druckniveau mit Druckluft beaufschlagt wird, wobei während der pneumatischen Dichtigkeitsmessung nach kurzzeitigem Öffnen eines Ablassventils ohne weitere Druckluftzufuhr ein zeitlicher Verlauf des Luftdrucks im Endoskop (2) gemessen wird, wobei die zeitliche Änderung des Luftdrucks am Ablassventil erfasst wird und in Abhängigkeit des zeitlichen Gradienten der zeitlichen Änderung des Luftdrucks die Dichtigkeit des Endoskops (2) oder die Undichtigkeit des Endoskops (2) ermittelt wird.

2. Verfahren nach Anspruch 1, **dadurch gekennzeichnet, dass** bei Überschreiten eines Gradientensollwerts für den zeitlichen Gradienten der zeitlichen Änderung des Luftdrucks die Dichtigkeit des Endoskops (2) festgestellt wird.

3. Verfahren nach Anspruch 1 oder 2, **dadurch gekennzeichnet, dass** bei Unterschreiten eines Gradientensollwerts für den zeitlichen Gradienten der zeitlichen Änderung des Luftdrucks die Undichtigkeit des Endoskops (2) festgestellt wird.

4. Verfahren nach einem der Ansprüche 1 bis 3, **dadurch gekennzeichnet, dass**, vorzugsweise ausschließlich, der zeitliche Anstieg des Drucks nach Öffnen des Auslassventils und/oder dessen zeitliche Änderung erfasst werden.

5. Verfahren nach einem der Ansprüche 1 bis 3, **dadurch gekennzeichnet, dass** das Ablassventil für eine Zeitdauer von weniger als 20 Sekunden, insbesondere weniger als 10 Sekunden oder 5 Sekunden, geöffnet wird.

## Claims

1. A method for processing an, in particular flexible, endoscope (2), wherein, for the implementation of leak testing the endoscope (2) the endoscope (2) to be processed is connected in an airtight manner to a compressed air hose, which is connectable or connected to a compressed air source, wherein for implementing a pneumatic leak test the endoscope (2) is subjected to compressed air up to a predetermined or predeterminable pressure level via the compressed air hose, wherein during the pneumatic leak measurement after brief opening of an outlet valve a temporal course of the air pressure in the endoscope (2) is measured without any further compressed air supply, wherein the temporal change of the air pressure at the outlet valve is measured and the tightness of the endoscope (2) or the leakage of the endoscope (2) is determined in dependency of the temporal gradient of the temporal change of the air pressure.

2. The method according to claim 1, **characterized in that**, in the event that a gradient set-point value for the temporal gradient of the temporal change of the air pressure is exceeded, the tightness of the endoscope (2) is determined.

3. The method according to claim 1 or 2, **characterized in that**, in the event that the gradient of the temporal change of the air pressure falls below a gradient set-point value, the leakage of the endoscope (2) is determined.

4. The method according to one of claims 1 to 3, **characterized in that**, preferably exclusively, the temporal increase of the pressure after the opening of the outlet valve is determined and/or the temporal change thereof is determined.

5. The method according to one of claims 1 to 3, **characterized in that** the outlet valve is opened for a duration of less than 20 seconds, in particular less than 10 seconds or less than 5 seconds.

## Revendications

1. Procédé pour préparer un endoscope (2), en particulier un endoscope souple, dans lequel un endoscope (2) à préparer est relié à un tuyau d'air comprimé de manière étanche à l'air comprimé afin de contrôler l'étanchéité de l'endoscope (2), lequel tuyau d'air comprimé peut être relié ou est relié à une source d'air comprimé, dans lequel de l'air comprimé est appliqué sur l'endoscope (2) par l'intermédiaire du tuyau d'air comprimé jusqu'à un niveau de pression prédéfini ou pouvant être prédéfini pour la réalisation d'un test d'étanchéité pneumatique, dans lequel pendant la mesure d'étanchéité pneumatique, après l'ouverture rapide d'un clapet de décharge sans apport d'air comprimé supplémentaire, une courbe temporelle de la pression de l'air dans l'endoscope (2) est mesurée, dans lequel la variation temporelle de la pression d'air est détectée au niveau du clapet de décharge et l'étanchéité de l'endoscope (2) ou le défaut d'étanchéité de l'endoscope (2) est déterminé en fonction du gradient temporel de la variation temporelle de la pression d'air.

2. Procédé selon la revendication 1, **caractérisé en ce que** l'étanchéité de l'endoscope (2) est établie lorsqu'une valeur de consigne de gradient pour le gradient temporel de la variation temporelle de la pression d'air est dépassée.

3. Procédé selon la revendication 1 ou 2, **caractérisé en ce que** le défaut d'étanchéité de l'endoscope (2) est établi lorsque le gradient de pression temporelle de la variation temporelle de la pression d'air chute sous une valeur de consigne de gradient.

4. Procédé selon l'une des revendications 1 à 3, **caractérisé en ce que** l'augmentation temporelle de la pression et/ou sa variation temporelle est déterminée, de préférence exclusivement, après l'ouverture du clapet de décharge.

5. Procédé selon l'une des revendications 1 à 3, **caractérisé en ce que** le clapet de décharge est ouvert pendant une durée inférieure à 20 secondes, en particulier inférieure à 10 secondes ou 5 secondes.
